# EUROPÄISCHE PATENTSCHRIFT

(11) **EP 1 003 584 B1**
(45) Veröffentlichungstag und Bekanntmachung des Hinweises auf die Patenterteilung: **29.10.2003**
(21) Anmeldenummer: 98949882.9
(22) Anmeldetag: 08.08.1998
(51) Int. Cl.: A61M 16/04

(54) **ENDOTRACHEAL- ODER TRACHEOTOMIETUBUS**
ENDOTRACHEAL OR TRACHEOTOMY TUBE
TUBE ENDOTRACHEAL OU DE TRACHEOTOMIE

(30) Priorität: 12.08.1997 DE 19734821
(43) Veröffentlichungstag der Anmeldung: 31.05.2000
(73) Patentinhaber: Tracoe Gesellschaft für Medizinische Bedarfsgegenstände MBH, 63263 Neu-Isenburg (DE)
(72) Erfinder: WALDECK, Franz, D-55268 Nieder-Olm (DE)
(74) Vertreter: Weber, Dieter, Dr. Dipl.-Chem.
(86) Internationale Anmeldenummer: DE9802341
(87) Internationale Veröffentlichungsnummer: WO99007428

(56) Entgegenhaltungen:
- US-A- 4 423 725
- US-A- 4 632 108
- US-A- 4 977 894

## Beschreibung

Die vorliegende Erfindung betrifft einen Endotracheal- oder Tracheotomietubus, dessen zentrales Lumen der Zu- und Abfuhr von Atemluft dient, mit einer den Tubus umfassenden Dichtmanschette (Cuff), einer Leitung für die Druckluftversorgung der Manschette, einer oberhalb der Manschette sich zur Außenseite des Tubus öffnenden Absaugleitung und einer zur Außenseite des Tubus oberhalb der Manschette mündenden Zuführleitung.

Ein entsprechender Endotrachealtubus ist aus dem US-Patent 5,582,167 bekannt. Bei dem bekannten Endotrachealtubus ist zusätzlich zu dem eigentlichen, das Lumen für das Atmen bzw. die Beatmung definierenden Tubus noch ein weiterer, den Tubus umfassender Schlauch aus einem dünnwandigen, nachgiebigen Material vorgesehen. Diese Schlauchhülle hat einen um 1 bis 8 mm größeren Innendurchmesser als es dem Außendurchmesser des eigentlichen Beatmungstubus entspricht, wobei sich in der Nähe der Manschette das Ende mit dem kleineren Durchmesser des Umhüllungsschlauches befindet, so daß zwischen der Außenwand des Tubus und der Innenwand der Schlauchhülle ein ringförmiger Kanal gebildet wird, der dicht oberhalb der Manschette endet und zum Absaugen von Sekret und dergleichen dient. Im unteren Bereich des Tubus, d.h. unmittelbar oberhalb der Manschette, sind zu diesem Zweck Abstandhalter für die Schlauchhülle vorgesehen, während im oberen, weiteren Teile keine entsprechenden Abstützeinrichtungen vorgesehen sind.

Ein wesentlicher Nachteil einer solchen Ausgestaltung eines Endotracheal- oder Tracheotomietubus liegt in der Nachgiebigkeit der äußeren Schlauchhülle. Da beim Absaugen zwischen der Außenwand des eigentlichen Tubus und der Schlauchhülle ein Unterdruck herrscht, besteht in hohem Maße die Gefahr, daß die äußere,nachgiebige Schlauchhülle kollabiert und sich an die Außenwand des Tubus anlegt. Es versteht sich, daß der vorgesehene Ringkanal zwischen der Außenwand des Tubus und der Innenwand der äußeren Schlauchhülle dann die vorgesehene Absaugfunktion nicht erfüllen kann. Auch die im unteren Bereich und unmittelbar oberhalb der Manschette vorgesehenen Abstandhalter zwischen Tubus und Schlauchhülle können dies Problem allenfalls geringfügig abmildern, jedoch nicht vollständig beseitigen.

Darüberhinaus mag der ringförmige Spalt zwischen Tubus und äußerer Schlauchhülle einen im Prinzip ausreichenden Gesamtquerschnitt haben, jedoch ist dieser Ringspalt ausgesprochen schmal, so daß Schleimpfropfen nicht oder nur mühsam abgesaugt werden können und relativ leicht den engen Spalt zwischen Tubus und Schlauchhülle verstopfen.

Das Absaugen oberhalb der Manschette hat vor allem den Zweck, sich dort ansammelnde Flüssigkeit, die Bakterien und sonstige Krankheitserreger enthält, welche in dem feuchten und warmen Milieu, das in der Luftröhre herrscht, ansonsten einen hervorragenden Nährboden finden, möglichst vollständig abzusaugen und zu entfernen, damit der intubierte bzw. eine Tracheotomiekanüle tragende Patient nach Möglichkeit keine Infektion der Bronchien oder der Lungen erleidet.

Wenn aber das Absaugen aufgrund der kollabierenden äußeren Schlauchhülle nur unzureichend erfolgt, so ist auch der angestrebte Infektionsschutz durch das Absaugen nur sehr unvollkommen.

Gegenüber diesem Stand der Technik liegt der vorliegenden Erfindung die Aufgabe zugrunde, einen Endotracheal- oder Tracheotomietubus zu schaffen, welcher eine sichere Absaugfunktion gewährleistet, d.h. mit welchem auch zähflüssiger Schleim oder größere Schleimpfropfen, die sich möglicherweise oberhalb der Manschette in der Luftröhre eines Patienten angesammelt haben, zuverlässig abgesaugt werden können.

Diese Aufgabe wird dadurch gelöst, daß die Wand des Tubus in einem Sektor ihres Umfanges einen verdickten, hohlen Abschnitt aufweist, dessen Hohlraum einen Absaugkanal definiert.

Indem also der Absaugkanal in die Wand des Tubus integriert wird, wozu allerdings auch zur Erzielung eines ausreichenden Absaugquerschnittes dieser Wandabschnitt verdickt sein muß, erhält man einen relativ stabilen, sich nicht ohne weiteres verformenden und damit nicht kollabierenden Absaugkanal, dessen minimaler Durchmesser problemlos größer gestaltet werden kann als die minimale Breite eines ringförmig den Tubus umfassenden Kanales.

Zwar erfolgt die Verdickung eines Wandabschnittes in einem Sektor (bezogen auf die Umfangsrichtung) des Tubus zu Lasten des Querschnittes des Lumens, diese Verringerung des Lumenquerschnittes kann jedoch vergleichsweise gering in der Größenordnung von zum Beispiel 10% gehalten werden, wobei immer noch der Durchmesser des Absaugkanales wesentlich größer gestaltet werden kann als die Spaltbreite eines den Tubus umgebenden Ringspaltes. Falls erwünscht, kann man zur Wiederherstellung des vollen Lumenquerschnittes den Außendurchmesser und damit auch den Lumendurchmesser des Tubus geringfügig vergrößern. Dabei wird eine Verringerung des Lumenquerschnittes aufgrund des verdickten Wandabschnittes um zum Beispiel etwa 10% schon durch eine Durchmesservergrößerung des Lumens und des gesamten Tubus um nur etwa 3 - 4% bereits vollständig kompensiert. Dagegen muß, ausgehend von einem vorgegebenen Lumenquerschnitt, der Außendurchmesser eines Tubus um 20% vergrößert werden, wenn die Spaltbreite zwischen einer äußeren Schlauchhülle und der Außenwand des Tubus ein Zehntel des Lumendurchmessers nicht unterschreiten soll.

Bevorzugt ist eine Ausführungsform der Erfindung, bei welcher der Sektor des verdickten Wandabschnittes weniger als 120°, vorzugsweise weniger als 90° vom Umfang des Tubus (im Querschnitt gesehen) umfaßt.

Der minimale Durchmesser des Absaugkanals sollte mindestens 1,5 mm, vorzugsweise mindestens 2 mm betragen. Dabei kann man zum Beispiel bei einem Tubusaußendurchmesser von 12 mm und einer normalen Tubuswandstärke außerhalb des verdickten Bereiches von etwa 1 mm ohne weiteres in einem verdickten Wandabschnitt, der sich über einen Sektor von weniger als 90° erstreckt, einen Absaugkanal mit einem minimalen Durchmesser von 3 mm unterbringen, wobei der freie Lumenquerschnitt nur um 10% reduziert wird. Will man bei einer solchen Ausführungsform den vollen Lumenquerschnitt (im Vergleich zu einem Tubus ohne Absaugkanal) erhalten, so braucht man lediglich, unter Beibehaltung der Wandstärke von 1 mm, den Tubusaußendurchmesser auf zum Beispiel 12,4 mm zu vergrößern, unter Beibehaltung eines kreisförmigen Absaugkanales mit einem Durchmesser von 3 mm. Der verdickte Wandabschnitt kann dann unter Einbeziehung des Absaugkanales eine maximale Dicke von 3,5 bis 4 mm haben, wobei sich zwar für den Absaugkanal selbst kurze Wandabschnitte zur Außenseite und zur Innenseite des Tubus bilden, die eventuell nur zwischen 0,2 und 0,5 mm dick sind, die jedoch sehr schnell in dickere Wandabschnitte des Tubus übergehen und so der Stabilität des Tubus insgesamt keinen Abbruch tun.

Wahlweise kann der Querschnitt des Absaugkanales kreisförmig sein, er kann aber auch oval bzw. elliptisch gestaltet werden, wobei in letzterem Fall das Verhältnis von langer zu kurzer Achse des elliptischen Querschnittes einen Wert von 2 bis 3 möglichst nicht überschreiten sollte, weil ansonsten entweder der minimale Durchmesser in Form der kurzen Achse des elliptischen Querschnittes zu klein oder aber der Anteil des Absaugkanales und damit des verdickten Wandabschnittes am Gesamtquerschnitt des Tubus zu groß wird. Ansonsten kann jedoch ein elliptischer Querschnitt des Absaugkanales im Hinblick auf maximale Ausnutzung des zur Verfügung stehenden Querschnittes bei gleichzeitiger Stabilität und Flexibilität des Tubus und seiner Wandabschnitte die optimale Lösung für die der Erfindung zugrunde liegende Aufgabe sein.

Zweckmäßigerweise sind in der bevorzugten Ausführungsform der Erfindung die Absaug- und die Zufuhrleitung auf diametral gegenüberliegenden Seiten des Tubus vorgesehen. Die Absaugöffnung bzw. Mündung des Absaugkanales am distalen Ende desselben liegt vorzugsweise unmittelbar oberhalb des Randes der Manschette.

Auch die Mündung des Zuführkanales, der vorzugsweise für die Zufuhr einer Spül- und/oder Desinfektionsflüssigkeit oder für Medikamente vorgesehen ist, kann in der gleichen axialen Höhe, d.h. relativ dicht oberhalb des oberen Randes der Manschette vorgesehen sein, kann aber auch axial nach oben versetzt sein.

Dabei ist der Querschnitt des Absaugkanals wesentlich größer als der Querschnitt der Zuführleitung und beträgt zum Beispiel das Fünf- bis Fünfzehnfache des letzteren. Der Querschnitt des Absaugkanals beträgt seinerseits zwischen 5 und 15% des Lumens des Tubus.

Zweckmäßigerweise sind sowohl die Zuführleitung als auch die Druckversorgungsleitung für die Manschette in die Wand des Tubus integriert. Da die beiden letztgenannten Leitungen nur einen relativ geringen Querschnitt haben müssen, denn in einem Fall wird Luft oder ein anderes Gas zum Aufblasen der Manschette zugeführt, im anderen Fall kann eine Spülflüssigkeit niedriger Viskosität verwendet werden, können diese Kanäle bzw. Leitungen mit einem typischen Durchmesser in der Größenordnung von 0,5 bis 0,8 mm ohne weiteres in die Tubuswand integriert werden, die hierfür nicht unbedingt verdickt zu werden braucht. Wahlweise kann jedoch in Analogie zu dem verdickten Abschnitt für die Absaugleitung die Tubuswand auch im Bereich der Zuführleitung für Spülflüssigkeit oder der Druckversorgungsleitung für die Manschette etwas verdickt sein, wenn auch in erheblich geringerem Maße als im Falle der Absaugleitung.

Gemäß einer weiteren Ausführungsform der vorliegenden Erfindung ist vorgesehen, daß ein zusätzlicher Absaugkanal vorgesehen ist, welcher in der Nähe des distalen Endes des Tubus, und zwar jenseits der Manschette, mündet. Über diesen zusätzlichen Absaugkanal kann dann Flüssigkeit bzw. Sekret aus der Luftröhre des Patienten abgesaugt werden, welches sich noch unterhalb der Manschette bzw. auf der dem äußeren Adapteranschluß des Tubus abgewandten Seite der Manschette ansammelt.

Auch dieser Absaugkanal ist vorzugsweise in die Wand des Tubus integriert, und zwar nach Möglichkeit in der Nähe des ersten Absaugkanals, welcher oberhalb der Manschette mündet und nach Möglichkeit auch in demselben verdickten Wandabschnitt. Dabei könnte eventuell auch einer der beiden Absaugkanäle ein getrennt auf die Außenwand des Tubus aufgeklebter Absaugkanal sein.

Außerdem ist es noch zweckmäßig, wenn die Mündungen des Absaugkanals bzw. der Absaugkanäle eine Einrichtung zum Freihalten der Mündungsöffnungen aufweisen, wie zum Beispiel einen oder mehrere, gegebenenfalls auch kreuzförmig angeordnete Stege oder dergleichen, welche von der Kanalöffnung etwas hervorstehen und so zwar mit der Wand der Luftröhre in Berührung treten können, gleichzeitig jedoch die Luftröhrenwand so in Abstand von der Mündung bzw. Kanalöffnung halten, daß mindestens an den Stegen oder dergleichen vorbei noch Zugang zu dem Kanal besteht. Alternativ könnte auch eine vorstehende Siebabdeckung an der Mündung des Absaugkanals bzw. der Absaugkanäle vorgesehen werden.

Weitere Vorteile, Merkmale und Anwendungsmöglichkeiten der vorliegenden Erfindung ergeben sich aus der folgenden Beschreibung einer bevorzugten Ausführungsform und der dazugehörigen Figuren. Es zeigen:
- Figur 1: einen Längsschnitt durch eine erfindungsgemäße Tracheotomiekanüle,
- Figur 2: einen Querschnitt durch eine Tracheotomiekanüle mit einem Schnitt entlang der Linie II-II in Figur 1,
- Figur 3: einen Querschnitt durch eine Tracheotomiekanüle mit einem Schnitt entlang der Linie III-III in Figur 1,
- Figur 4: einen Querschnitt durch eine Tracheotomiekanüle mit einem Schnitt entlang der Linie IV-IV in Figur 1,
- Figur 5: einen Längsschnitt durch eine weitere Ausführungsform der erfindungsgemäße Tracheotomiekanüle,
- Figur 6: einen Querschnitt durch eine Tracheotomiekanüle nach Figur 5 mit einem Schnitt entlang der Linie Vi-VI in Figur 5,
- Figur 7: einen Querschnitt durch eine Tracheotomiekanüle nach Figur 5 mit einem Schnitt entlang der Linie VII-VII in Figur 5,
- Figur 8: einen Querschnitt durch eine Tracheotomiekanüle nach Figur 5 mit einem Schnitt entlang der Linie VIII-VIII in Figur 5,
- Figur 9: einen Querschnitt durch eine Tracheotomiekanüle nach Figur 5 mit einem Schnitt entlang der Linie IX-IX in Figur 5 und
- Figur 10: einen Querschnitt durch eine Tracheotomiekanüle nach dem Stand der Technik

Die Darstellungen in den Figuren sind nur bedingt maßstabsgetreu und stellen insoweit keine Festlegungen dar, auch wenn in der Praxis einige der dargestellten Aspekte durchaus auch in den relativen Maßen identisch übernommen werden könnten.

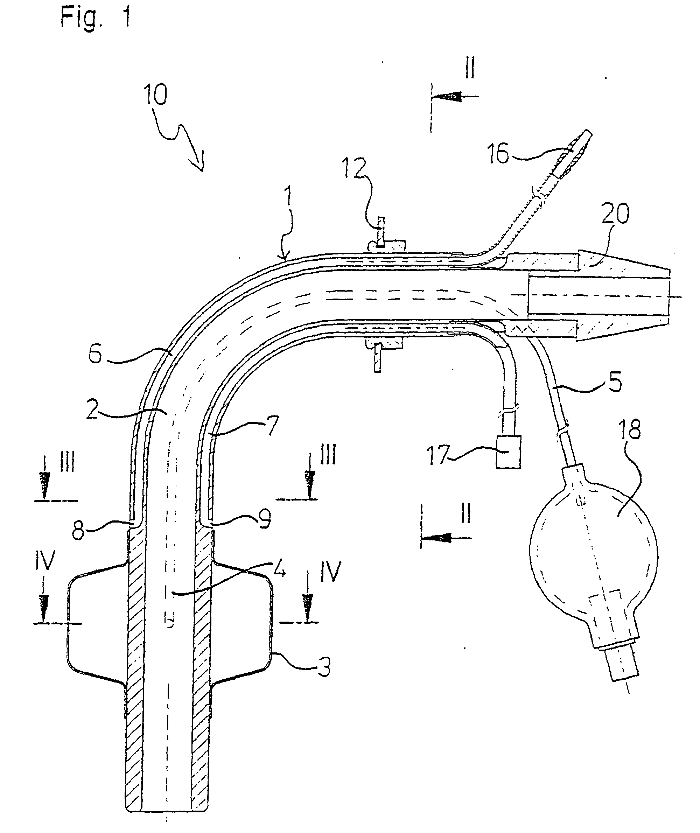

in Figur 1 erkennt man einen insgesamt mit 10 bezeichneten Tracheotomietubus bzw. eine Tracheotomiekanüle, die dafür vorgesehen ist, nach einem entsprechenden operativen Schnitt durch den Hals eines Patienten in dessen Luftröhre eingeführt zu werden. Die Tracheotomiekanüle besteht aus einem Tubus 1, einem sogenannten Schild 12, welches am Hals des Patienten anliegt und dadurch die Lage der Kanüle definiert, einer Abdichtmanschette 3 und einem genormten Adapter (20) am Eingangsende der Kanüle für den Anschluß an ein Narkoseoder Beatmungsgerät. Der untere Abschnitt der Kanüle 10 mit der Manschette 3 befindet sich in der Luftröhre eines Patienten, im allgemeinen unterhalb des Kehlkopfes und der Stimmbänder. Beim Einführen der Kanüle befindet sich die Manschette 3 in einem nicht-aufgeblasenen, eng an dem Tubus 1 anliegenden Zustand und wird anschließend über eine Leitung 4 und deren äußeren Anschluß 5 auf einen leichten Überdruck von typischerweise 15 - 20 mm Hg aufgebläht, um in der Luftröhre des Patienten eine Abdichtung und eine Fixierung des Tubus zu bilden. Ein Endotrachealtubus unterscheidet sich von einer solchen Tracheotomiekanüle im wesentlichen nur dadurch, daß er wesentlich länger ist und nicht den Schild 12 aufweist, da ein Endotrachealtubus im allgemeinen durch den Mund oder die Nase eines Patienten in die Luftröhre eingeführt wird.

Ein Ventil und ein Druckregel- bzw. Überwachungssystem für die Manschette 3 werden in Figur 1 durch den Ballon 18 und damit verbundene Elemente repräsentiert. Ein Problem bei längeren Intubationen und beim Tragen von Tracheotomiekanülen liegt oftmals darin, daß sich in der Luftröhre eines Patienten unmittelbar oberhalb der Manschette 3 und außerhalb des Tubus 1 Flüssigkeit und Schleim ansammelt und aufgrund des feuchten und warmen Klimas, welches notwendigerweise in diesem Bereich herrscht, einen idealen Nährboden für Bakterien und andere Krankheitserreger bilden. Diese können unter Umständen durch Druckschwankungen in der Manschette 3 oder durch Bewegungen des Patienten auch an der Manschette 3 vorbeitreten, oder aber beim zwischenzeitlichen Herausnehmen des Tubus in die Bronchien und die Lunge des Patienten eindringen und verursachen dadurch oftmals schwere Komplikationen, wie Lungenentzündungen und andere Krankheiten, von denen zum Beispiel nach Operationen ein großer Anteil der längerfristig intubierten Patienten befallen wird.

Zur Beseitigung oder Abmilderung dieses Problems ist in den in Figur 1 dargestellten Tubus 1 ein Absaugkanal 6 integriert, mit einem äußeren Absauganschluß 16 und einer inneren Absaugöffnung 8, die unmittelbar oberhalb der Manschette die Verbindung von der äußeren Umgebung des Tubus 1 zu dem Absaugkanal 6 herstellt. Durch diesen Absaugkanal 6 können Schleim und Flüssigkeit, die sich in der Luftröhre oberhalb der Manschette 3 ansammeln, ohne weiteres abgesaugt und entfernt werden, dadurch werden auch die darin enthaltenen Bakterien und sonstigen Krankheitskeime mit entfernt und in ihrer Anzahl erheblich reduziert und es wird den verbleibenden Krankheitserregern auch der größte Teil des ansonsten vorhandenen Nährmediums entzogen, so daß die Zahl und Dichte der Krankheitskeime in diesem Bereich vergleichsweise gering ist und das Immunsystem auch eines geschwächten Patienten die Abwehr dieser verbleibenden Krankheitskeime bewältigen kann.

Zusätzlich ist in dem Längsschnitt gemäß Figur 1 auch noch ein Spülkanal 7 zu erkennen mit einem äußeren Anschluß 17 und einer inneren Öffnung 9, die sich ebenfalls zur Außenseite des Tubus 1 öffnet. Durch diesen Kanal 7 kann in den den Tubus oberhalb der Manschette 3 umgebenden Bereich eine Flüssigkeit eingespritzt werden, die dann ebenfalls durch die Öffnung 8 und den Absaugkanal 6 wieder abgesaugt wird. Hierdurch kann die Zahl der Krankheitserreger in diesem Bereich noch weiter erheblich vermindert werden und außerdem ist es möglich, der Spülflüssigkeit bakterizid wirkende Stoffe oder Antibiotika beizumengen, um so vorhandene Krankheitskeime gegebenenfalls abzutöten oder deren Vermehrung zu verhindern. Die Kombination von Spülung und Absaugen kann das Krankheitsrisiko des Patienten damit erheblich herabsenken. Dabei ist es im übrigen auch möglich, die Öffnung 9 des Spülkanals 7 etwas weiter oben als die Absaugöffnung münden zu lassen, wenn es zum Beispiel erwünscht ist, einen etwas längeren Abschnitt oberhalb der Manschette 3 und um den Tubus 1 herum mit der Spülflüssigkeit zu beaufschlagen. Das Absaugen kann auch zeitlich versetzt zu der Zugabe der Spülflüssigkeit erfolgen, indem zum Beispiel zunächst die vorhandene Flüssigkeit bzw. Schleim durch die Öffnung 8 abgesaugt wird, dann durch die innere Leitung 7 Spülflüssigkeit zugeführt und gegebenenfalls auch gleichzeitig abgesaugt wird, wobei dann das Absaugen auch unterbrochen werden kann, um den Pegel der Spülflüssigkeit bis zu einem gewünschten Niveau ansteigen zu lassen, damit ein etwas größerer Bereich der Luftröhre gereinigt und von Krankheitserregern befreit wird bzw. deren Konzentration in diesem Bereich erheblich herabgesetzt wird. Auch Medikamente, die in diesem Bereich von der Wand der Luftröhre aufgenommen werden und so in die Blutbahn des Patienten gelangen, können durch den Spülkanal 7 zugeführt werden.

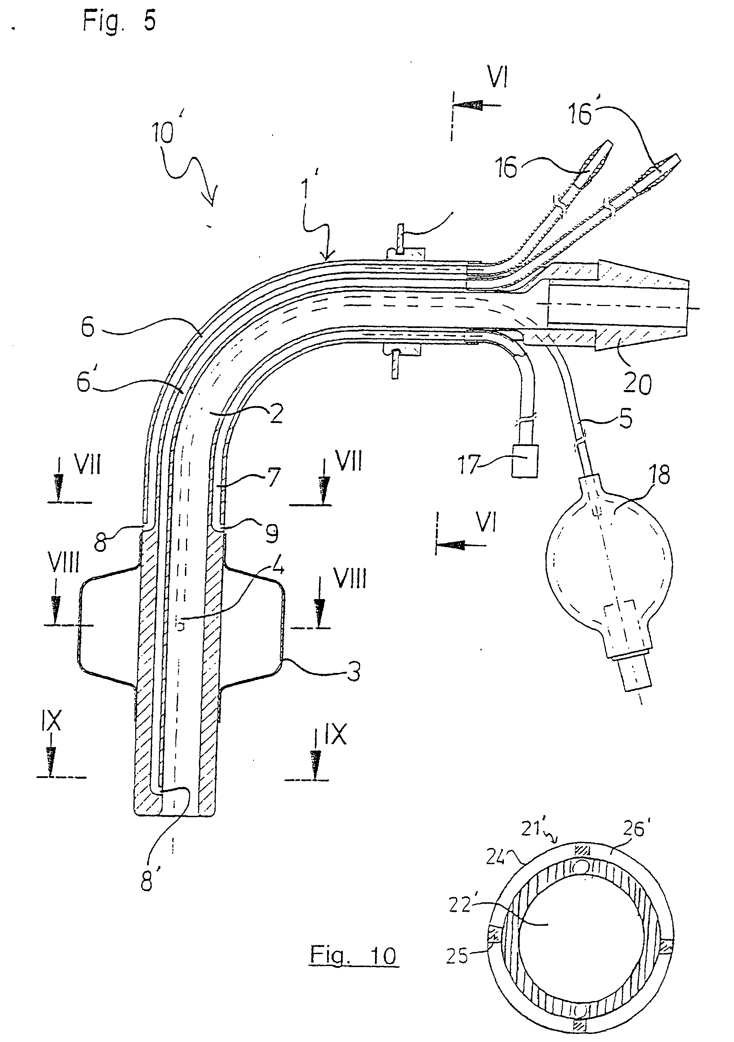

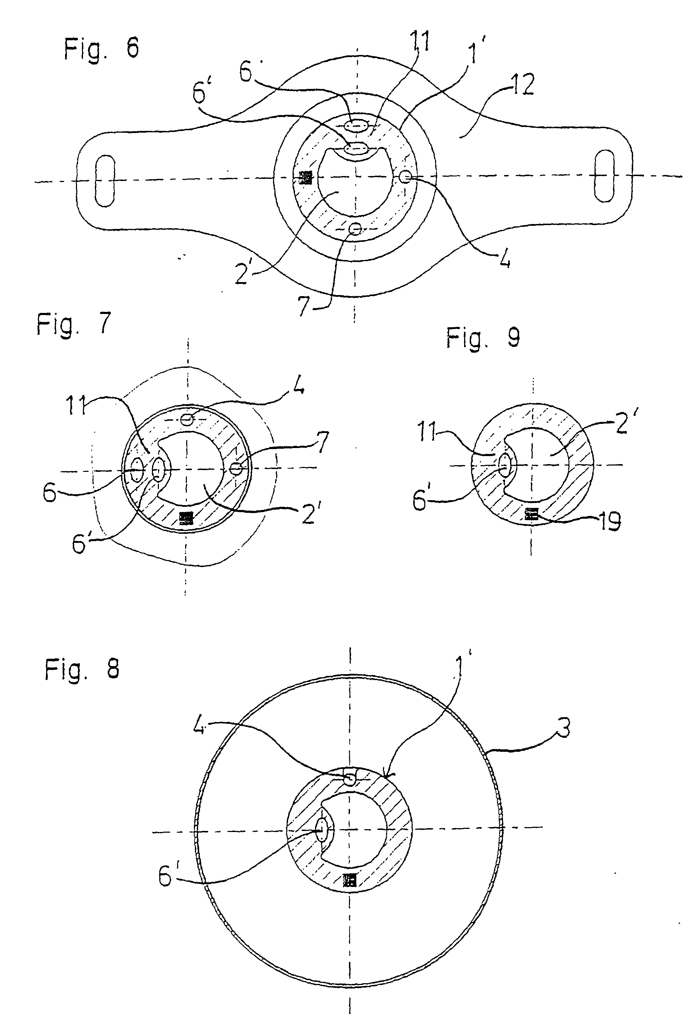

Neben dem Absaugkanal 6 kann in einer besonderen Ausführungsform der Erfindung, wie sie in den Figuren 5 bis 8 dargestellt ist, auch noch ein zweiter, unterhalb der Manschette und in der des distalen Endes der Kanüle mündender Absaugkanal 6' vorgesehen sein. Vorzugsweise ist dann der letztere in die Tubuswand integriert, während der ursprüngliche Absaugkanal 6, der nach wie vor vorhanden ist und oberhalb der Manschette 2 mündet, wahlweise ebenfalls in die Tubuswand integriert oder außen auf die Tubuswand aufgeklebt ist.

Der Kanal 4 zum Aufpumpen bzw. Druckregulieren des Druckes in der Manschette 3 kann ebenso wie der Spülkanal 7 in die Wand des Tubus 1 integriert sein, ist jedoch gegenüber den beiden dargestellten Kanälen 7 und 6 um 90° versetzt und daher in der Darstellung gemäß Figur 1 und 5 nur durch gestrichelte Linien angedeutet.

Der Patient atmet durch das zentrale Lumen 2 des Tubus 1 bzw. wird durch dieses Lumen 2 beatmet, falls sein Zustand dies erfordert. Je nach Anwendungsfall hat der Tubus 1 sehr unterschiedliche Abmessungen und insbesondere auch unterschiedliche Durchmesser, da Kinder zum Beispiel einen wesentlich kleineren Tubus und auch ein kleineres Lumen benötigen als zum Beispiel körperlich große erwachsene Personen. Wesentlich ist selbstverständlich, daß das zentrale Lumen 2 einen genügend großen Querschnitt hat, so daß der Patient problemlos und ohne übermäßige Anstrengungen selbst atmen kann oder aber entsprechend beatmet werden kann. Andererseits setzen jedoch die konkreten anatomischen Gegebenheiten bei einem Patienten dem Außendurchmesser des Tubus 1 bzw. 1' Grenzen. Insofern ist es wesentlich, daß die Anordnung und auch die Größe der Kanäle 4, 6, 6' und 7 so gewählt werden, daß bei gegebenem Lumenquerschnitt der Außendurchmesser des Tubus nicht allzu groß wird. Dabei ist jedoch zu berücksichtigen, daß der manchmal etwas zähflüssige Schleim, der sich in der Luftröhre eines Patienten bilden kann, nicht ohne weiteres durch kleine Querschnitte abgesaugt werden kann, die dann allzu leicht verstopfen würden.

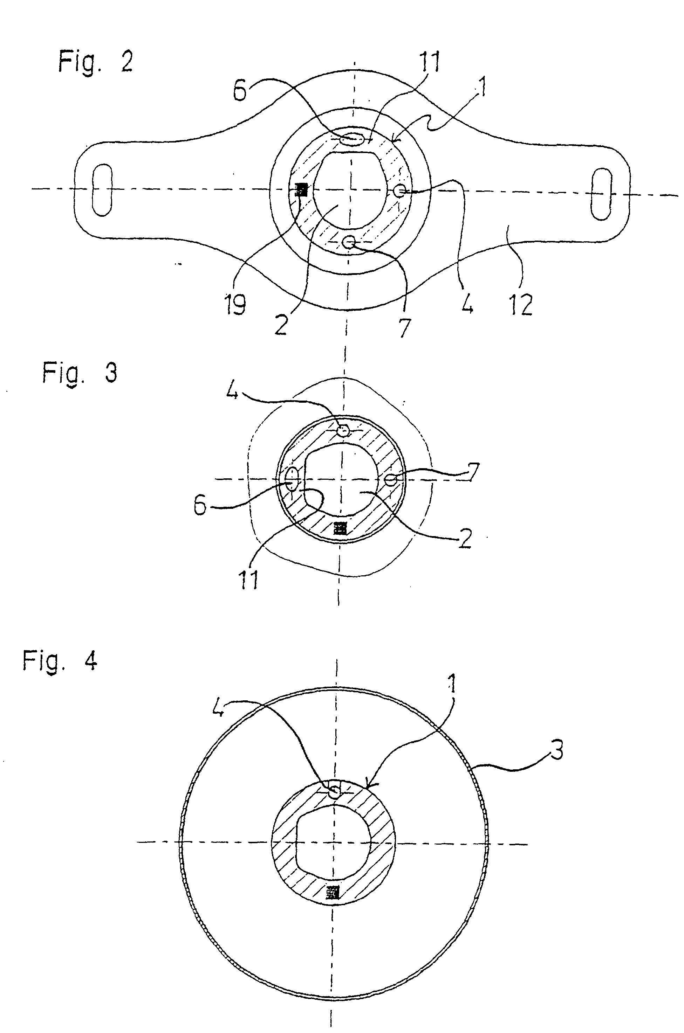

Wie man sowohl anhand des Längsschnittes der Figur 1, vor allem aber anhand des Querschnittes gemäß Figur 2 erkennen kann, sieht die vorliegende Erfindung einen verdickten Wandabschnitt 11 vor, der hohl ausgebildet ist und einen Kanal 6 zum Absaugen aufweist, dessen Querschnitt hinreichend groß ist, um auch zähflüssigen Schleim oder Schleimpfropfen, die sich in der Luftröhre eines Patienten oberhalb der Manschette 3 gebildet haben, absaugen zu können. Konkret hat der Absaugkanal 6 einen in etwa kreisförmigen oder ovalen Querschnitt, der größenordnungsmäßig etwa 10% des Querschnitts des Lumens 2 ausmacht. Wie man erkennt, ist der Querschnitt des Lumens 2 bzw. 2' durch den verdickten Wandabschnitt 11 etwas verringert, jedoch beträgt die Verringerung des Lumenquerschnitts bei den konkret in Figur 2 dargestellten Querschnittsverhältnissen nur wenige % gegenüber einem kreisförmigen Lumenquerschnitt mit demselben Durchmesser, jedoch ohne den verdickten Wandabschnitt 11. Falls es jedoch erforderlich ist, den vollen Lumenquerschnitt beizubehalten, der ohne den verdickten Wandabschnitt 11 vorhanden wäre, so genügt es, den Gesamtdurchmesser des Tubus 1 um etwa 3 - 4% zu vergrößern, so daß sich auch der Innendurchmesser des Lumens 2 um 3 - 4% vergrößert, was zur Vergrößerung des Lumenquerschnittes in der Größenordnung von 10 - 15% führt, so daß dann der von dem verdickten Wandabschnitt 11 beanspruchte Querschnittsanteil durch die geringfügige Vergrößerung des Gesamtdurchmessers kompensiert wird. Der Spülkanal 7 liegt dem Absaugkanal 6 diametral gegenüber und hat einen erheblich kleineren Querschnitt, so daß er innerhalb der normalen Wandstärke der Wand 13 des Tubus 1 Platz findet, ebenso wie auch der Luftdruckkanal 4 für das Aufblasen der Manschette 3. Die Spülflüssigkeit kann gegebenenfalls mit entsprechend niedriger Viskosität ausgewählt werden und kann bei Bedarf auch mit erhöhtem Druck durch den Kanal 7 eingepreßt oder infundiert werden, so daß es ausreicht, wenn der Kanal 7 einen Durchmesser in der Größenordnung von 0,5 mm hat. Ein entsprechender Durchmesser ist auch für den Druckluftkanal 4 ohne weiteres ausreichend. Dagegen ist der beim Absaugen zu erzeugende Unterdruck auf die Größenordnung des normalen Luftdruckes beschränkt und die Flüssigkeit bzw. der Schleim, der sich möglicherweise in der Luftröhre eines Patienten oberhalb der Manschette 3 ansammelt, hat möglicherweise eine relativ hohe Viskosität, so daß ein entsprechend größerer Querschnitt für den Absaugkanal 6 benötigt wird. Der verdickte Wandabschnitt 11 liefert dabei gleichzeitig eine ausreichende Stabilität für den Kanal 6, so daß dieser bei einer Druckdifferenz in der Größenordnung von 1 Bar auf keinen Fall kollabiert. Anhand der verschiedenen Querschnittdarstellungen in den Figuren 2 - 4 und 6 - 9, die Schnitte in verschiedenen axialen Positionen der Tracheotomietuben der ersten bzw. zweiten Ausführungsformen zeigen, erkennt man deutlich, bis zu welchen axialen Positionen die einzelnen Kanäle reichen und wie sich der Querschnitt der einzelnen Kanäle relativ zueinander und zum zentraklen Lumen verhält, auch wenn, wie bereits erwähnt, die relativen und absoluten Maße der einzelnen Kanäle durchaus von den dargestellten Maßen abweichen können.

Zum Vergleich ist in Figur 10 ein aus dem Stand der Technik bekannter Endotrachealtubus im Querschnitt dargestellt, der im Prinzip denselben Außendurchmesser haben muß wie der Tubus 1 oder 1' gemäß den Figuren 1-9 der vorliegenden Erfindung. Dabei wird ein Absaugkanal 26 durch einen Ringraum definiert, der außerhalb des Tubus 21 durch eine Schlauchhülle oder Rohrhülle 24 definiert wird. Bei einer solchen Schlauchhülle 24 besteht jedoch im Falle des Absaugens die Gefahr, daß diese Schlauchhülle 24 kollabiert und sich an die Wand des Tubus 21 anlegt und dadurch den Querschnitt des Kanales 26 erheblich reduziert oder sogar vollständig verschließt. Zwar mag der Gesamtquerschnitt des Absaugkanales 26 sogar größer sein als der Gesamtquerschnitt des Absaugkanales 6, jedoch liegt der Absaugkanal 26 nur in Form eines schmalen Ringkanales 26 vor, dessen Breite (in radialer Richtung) nur etwa ein Drittel des Durchmessers des Absaugkanales 6 beträgt, so daß zähflüssiger Schleim nur sehr schwer durch diesen Kanal 26 abgesaugt werden kann. Abstandhalter mögen zwar dazu dienen, ein Berühren der Schlauch- oder Rohrhülle 24 mit der Außenwand des Tubus 21 zu verhindern, jedoch gelingt dies nur, wenn die äußere Hülle 24 aus einem hinreichend steifen Material besteht und auch dann bleibt die radiale Breite des Ringspaltes 26 nach wie vor sehr klein gegen den Durchmesser des Absaugkanales 6 gemäß Figur 2.

Der Vergleich mit dem Stand der Technik gemäß Figur 10 zeigt im übrigen, daß der Querschnitt des zentralen Lumens 2 im Falle der vorliegenden Erfindung bei gegebenem Außendurchmesser des Tubus 1 bzw. 1' wesentlich größer ist als bei dem bekannten Stand der Technik. Umgekehrt könnte bei gleichem Querschnitt des zentralen Lumens 2 der Außendurchmesser des Tubus 1, 1' im Vergleich zu dem Tubus 21 nach dem Stand der Technik verringert werden, wobei gleichzeitig die Fähigkeit zum Absaugen dennoch verbessert wäre.

## Patentansprüche

1. Endotracheal- oder Tracheotomietubus (10), dessen zentrales Lumen (2) der Zu- und Abfuhr von Atemluft dient, mit einer den Tubus (1) umfassenden, aufblasbaren Dichtmanschette (3), einer Leitung (4) für die Druckluftversorgung der Manschette (3), einer oberhalb der Manschette (3) sich zur Außenseite des Tubus (1) öffnenden Absaugleitung (6) und einer ebenfalls oberhalb der Manschette (3) zur Außerseite des Tubus (1) mündenden Zuführleitung (7), **dadurch gekennzeichnet, daß** die Wand (9) des Tubus (1) in einem Sektorbereich entlang ihres Umfanges einen verdickten, hohlen Abschnitt (11) aufweist, dessen innerer Hohlraum die Absaugleitung (6) bildet.

2. Endotracheal- oder Tracheotomietubus nach Anspruch 1, **dadurch gekennzeichnet, daß** der Sektor des verdickten Wandabschnittes (11), in einem Querschnitt des Tubus gesehen, weniger als 120°, vorzugsweise weniger als 90° umfaßt.

3. Endotracheal- oder Tracheotomietubus nach Anspruch 1 oder 2, **dadurch gekennzeichnet, daß** der minimale Durchmesser des Absaugkanales (6) mindestens 1,5 mm, vorzugsweise mindestens 2 mm beträgt.

4. Endotracheal- oder Tracheotomietubus nach einem der Ansprüche 1 bis 3, **dadurch gekennzeichnet, daß** der Absaugkanal (6) einen im wesentlichen kreisförmigen Querschnitt hat.

5. Endotracheal- oder Tracheotomietubus nach einem der Ansprüche 1 bis 3, **dadurch gekennzeichnet, daß** der Absaugkanal (6) einen ovalen bzw. elliptischen Querschnitt hat, wobei das Verhältnis von großer zu kleiner Achse des elliptischen Querschnittes vorzugsweise weniger als 2 beträgt.

6. Endotracheal- oder Tracheotomietubus nach einem der Ansprüche 1 bis 5, **dadurch gekennzeichnet, daß** die Mündungen des Absaugkanales (6) und des Zuführkanales (7) auf diametral gegenüberliegenden Seiten des Tubus (1) vorgesehen sind.

7. Endotracheal- oder Tracheotomietubus nach einem der Ansprüche 1 bis 6, **dadurch gekennzeichnet, daß** die Absaugöffnung des Absaugkanales (6) unmittelbar am oberen Rand der Manschette (3) angeordnet ist.

8. Endotracheal- oder Tracheotomietubus nach einem der Ansprüche 1 bis 7, **dadurch gekennzeichnet, daß** die Mündung der Zuleitung gegenüber der Mündung des Absaugkanales (6) axial versetzt ist.

9. Endotracheal- oder Tracheotomietubus nach einem der Ansprüche 1 bis 8, **dadurch gekennzeichnet, daß** sowohl die Druckleitung (4) für die Manschette (3) als auch die Zuführleitung (7) in die Wand (9) des Tubus (1) integriert sind.

10. Endotracheal- oder Tracheotomietubus nach einem der Ansprüche 1 bis 9, **dadurch gekennzeichnet, daß** der Querschnitt des Absaugkanales (6) sehr viel größer ist als der Querschnitt der Zuführleitung (7), und mindestens das Fünf- bis Fünfzehnfache des Zuführleitungsquerschnittes beträgt.

11. Endotracheal- oder Tracheotomietubus nach einem der Ansprüche 1 bis 10, **dadurch gekennzeichnet, daß** der Querschnitt des Absaugkanals (6) zwischen 3% und 15% des Lumenquerschnittes beträgt.

12. Tracheotomietubus nach einem der Ansprüche 1 bis 11, **dadurch gekennzeichnet, daß** Anschlußstutzen (16, 17) für den Absaugkanal (6) bzw. den Zuführkanal (7) am proximalen Ende des Tubus (1) vor einem Schild (8) des Tracheotomietubus (10) münden.

13. Tracheotomietubus nach einem der Ansprüche 1 bis 12, **dadurch gekennzeichnet, daß** ein zusätzlicher Absaugkanal (6') vorgesehen ist, welcher in der Nähe des distalen Endes des Tubus (1') jenseits der Manschette (2) mündet.

14. Tracheotomietubus nach Anspruch 13, **dadurch gekennzeichnet, daß** der zusätzliche Absaugkanal (6') in die Wand des Tubus integriert ist, während der oberhalb der Manschette mündende Absaugkanal (6) auf die Außenwand des Tubus aufgeklebt ist.

15. Tracheotomietubus nach einem der Ansprüche 1 bis 14, **dadurch gekennzeichnet, daß** die Mündung des Absaugkanals (6) bzw. der Absaugkanäle (6, 6') eine Einrichtung zum Freihalten der Kanalöffnung aufweist.

## Claims

1. An endotracheal or tracheotomy tube whose central lumen (2) serves for the feed and discharge of respiration air, comprising an inflatable sealing cuff (3) which embraces the tube (1), a conduit (4) for the pressure air supply to the cuff (3), a suction conduit (6) which opens above the cuff (3) to the outside of the tube (1), and a feed conduit (7) which also opens to the outside of the tube (1) above the cuff (3), **characterised in that** in a sector region along its periphery the wall (9) of the tube (1) has a thickened hollow portion (11) whose internal cavity forms the suction conduit (6).

2. An endotracheal or tracheotomy tube as set forth in claim 1 **characterised in that** the sector of the thickened wall portion (11), viewed in a cross-section of the tube, embraces less than 120°, preferably less than 90°.

3. An endotracheal or tracheotomy tube as set forth in claim 1 or claim 2 **characterised in that** the minimum diameter of the suction passage (6) is at least 1.5 mm, preferably at least 2 mm.

4. An endotracheal or tracheotomy tube as set forth in one of claims 1 through 3 **characterised in that** the suction passage (6) is of a substantially circular cross-section.

5. An endotracheal or tracheotomy tube as set forth in one of claims 1 through 3 **characterised in that** the suction passage (6) is of an oval or elliptical cross-section, wherein the ratio of long to short axis of the elliptical cross-section is preferably less than 2.

6. An endotracheal or tracheotomy tube as set forth in one of claims 1 through 5 **characterised in that** the mouth openings of the suction passage (6) and the feed passage (7) are provided on diametrally opposite sides of the tube (1).

7. An endotracheal or tracheotomy tube as set forth in one of claims 1 through 6 **characterised in that** the suction opening of the suction passage (6) is arranged directly at the upper edge of the cuff (3).

8. An endotracheal or tracheotomy tube as set forth in one of claims 1 through 7 **characterised in that** the mouth opening of the feed conduit is axially displaced with respect to the mouth opening of the suction passage (6).

9. An endotracheal or tracheotomy tube as set forth in one of claims 1 through 8 **characterised in that** both the pressure conduit (4) for the cuff (3) and also the feed conduit (7) are integrated into the wall (9) of the tube (1).

10. An endotracheal or tracheotomy tube as set forth in one of claims 1 through 9 **characterised in that** the cross-section of the suction passage (6) is very much larger than the cross-section of the feed conduit (7) and is at least between five and fifteen times the feed conduit cross-section.

11. An endotracheal or tracheotomy tube as set forth in one of claims 1 through 10 **characterised in that** the cross-section of the suction passage (6) is between 3% and 15% of the lumen cross-section.

12. A tracheotomy tube as set forth in one of claims 1 through 11 **characterised in that** connection portions (16, 17) for the suction passage (6) and the feed passage (7) respectively open at the proximal end of the tube (1) in front of a shield plate (8) of the tracheotomy tube (10).

13. A tracheotomy tube as set forth in one of claims 1 through 12 **characterised in that** there is provided an additional suction passage (6') which opens in the proximity of the distal end of the tube (1') beyond the cuff (2).

14. A tracheotomy tube as set forth in claim 13 **characterised in that** the additional suction passage (6') is integrated into the wall of the tube while the suction passage (6) which opens above the cuff is glued onto the outside wall of the tube.

15. A tracheotomy tube as set forth in one of claims 1 through 14 **characterised in that** the mouth opening of the suction passage (6) or the suction passages (6, 6') has a means for keeping the passage opening free.

## Revendications

1. Tube endotrachéal ou de trachéotomie (10) dont la lumière centrale (2) sert à amener et emmener l'air de respiration, lequel tube comporte un manchon d'étanchéité (3) gonflable et entourant le tube (1), une conduite (4) destinée à l'alimentation en air sous pression du manchon (3), une conduite d'aspiration (6) située au-dessus du manchon (3) et s'ouvrant vers l'extérieur du tube (1) et une conduite d'amenée (7) également située au-dessus du manchon (3) et s'ouvrant également vers l'extérieur du tube (1), **caractérisé en ce que** la paroi (9) du tube (1) présente, le long de sa périphérie au niveau d'un secteur, une section creuse renflée (11) dont l'espace intérieur forme la conduite d'aspiration (6).

2. Tube endotrachéal ou de trachéotomie selon la revendication 1, **caractérisé en ce que** le secteur formé par la section de paroi renflée (11) est inférieur à 120°, avantageusement inférieur à 90°, lorsque le tube est observé en coupe transversale.

3. Tube endotrachéal ou de trachéotomie selon la revendication 1 ou 2, **caractérisé en ce que** le diamètre minimum du canal d'aspiration (6) est d'au moins 1,5 mm, avantageusement d'au moins 2 mm.

4. Tube endotrachéal ou de trachéotomie selon l'une des revendications 1 à 3, **caractérisé en ce que** le canal d'aspiration (6) a une section sensiblement circulaire.

5. Tube endotrachéal ou de trachéotomie selon l'une des revendications 1 à 3, **caractérisé en ce que** le canal d'aspiration (6) a une section ovale ou elliptique, et le rapport entre le grand axe et le petit axe de la section elliptique est avantageusement inférieur à 2.

6. Tube endotrachéal ou de trachéotomie selon l'une des revendications 1 à 5, **caractérisé en ce que** l'embouchure du canal d'aspiration (6) et l'embouchure du canal d'amenée (7) sont prévues sur des côtés diamétralement opposés du tube (1).

7. Tube endotrachéal ou de trachéotomie selon l'une des revendications 1 à 6, **caractérisé en ce que** l'ouverture d'aspiration du canal d'aspiration (6) est agencé directement au niveau du bord supérieur du manchon (3).

8. Tube endotrachéal ou de trachéotomie selon l'une des revendications 1 à 7, **caractérisé en ce que** l'embouchure de la conduite d'amenée est décalée axialement par rapport à l'embouchure du canal d'aspiration (6).

9. Tube endotrachéal ou de trachéotomie selon l'une des revendications 1 à 8, **caractérisé en ce que** aussi bien la conduite sous pression (4), destinée au manchon (3), que la conduite d'amenée (7) sont intégrées dans la paroi (9) du tube (1).

10. Tube endotrachéal ou de trachéotomie selon l'une des revendications 1 à 9,**caractérisé en ce que** la section du canal d'aspiration (6) est très supérieure à la section de la conduite d'amenée (7), et est au moins égale à une valeur comprise entre cinq fois et quinze fois la section de la conduite d'amenée.

11. Tube endotrachéal ou de trachéotomie selon l'une des revendications 1 à 10, **caractérisé en ce que** la section du canal d'aspiration (6) est comprise entre 3% et 15% de la section de la lumière.

12. Tube de trachéotomie selon l'une des revendications 1 à 11, **caractérisé en ce que** le raccord (16, 17) destiné au canal d'aspiration (6) ou au canal d'amenée (7) débouche au niveau de l'extrémité proximale du tube (1) en avant d'un flasque (8) du tube de trachéotomie (10).

13. Tube de trachéotomie selon l'une des revendications 1 à 12, **caractérisé en ce qu'**il est prévu un canal d'aspiration supplémentaire (6') qui débouche à proximité de l'extrémité distale du tube (1') de l'autre côté du manchon (2).

14. Tube de trachéotomie selon la revendication 13, **caractérisé en ce que** le canal d'aspiration supplémentaire (6') est intégré dans la paroi du tube tandis que le canal d'aspiration (6), débouchant au-dessus du manchon, est collé sur la paroi extérieure du tube.

15. Tube de trachéotomie selon l'une des revendications 1 à 14, **caractérisé en ce que** l'embouchure du canal d'aspiration (6) ou des canaux d'aspiration (6, 6') comporte un dispositif de protection de l'ouverture de canal.
